# EUROPEAN PATENT APPLICATION

(11) **EP 1 683 494 A1**
(43) Date of publication of application: **26.07.2006**
(21) Application number: 05290344.0
(22) Date of filing: 16.02.2005
(51) Int. Cl.: A61B 17/12

(54) **Vasoocclusive article**

(30) Priority: 24.01.2005 JP 2005015723
(71) Applicant: Medico's Hirata Inc., Osaka-shi, Osaka 530-0003 (JP)
(72) Inventor: Satake, Mitsuo, Tokyo 104-0045 (JP); Moriyama, Noriyuki, Tokyo 104-0045 (JP); Yokogawa, Tooru, Medico's Hirata Inc., Osaka-shi Osaka 550-0002 (JP); Saeki, Toshio, Medico's Hirata Inc., Osaka-shi Osaka 550-0002 (JP); Seki, Keiichi, Medico's Hirata Inc., Osaka-shi Osaka 550-0002 (JP); Miyata, Akitaka, Medico's Hirata Inc., Osaka-shi Osaka 550-0002 (JP)
(74) Representative: Peaucelle, Chantal

(57) **Abstract**

Poly(L-lactic acid) (200,000 in weight average molecular weight and 175° C in melting point) is melt-spun at 200° C and drawn to obtain a single filament having a diameter of 0.05 mm. Two lengths of the filament are twisted to prepare a filamentous substance, which is cut to a length of 7 cm to obtain a vasoocclusive article 1 in the form of a single line. The occlusive article is free of the problems conventionally experienced.

## Description

### BACKGROUND OF THE INVENTION

### (a) Field of the Invention

The present invention relates to articles for use in blood vessels of patients for occluding the blood vessel, and more particularly to an occlusive article made from a material absorbable by the living body. The vasoocclusive article is used for the occlusion of blood vessels, emergency arresting of internal hemorrhage, treatment of aneurysms or embolization therapy of cancers.

### (b) Description of the Prior Art

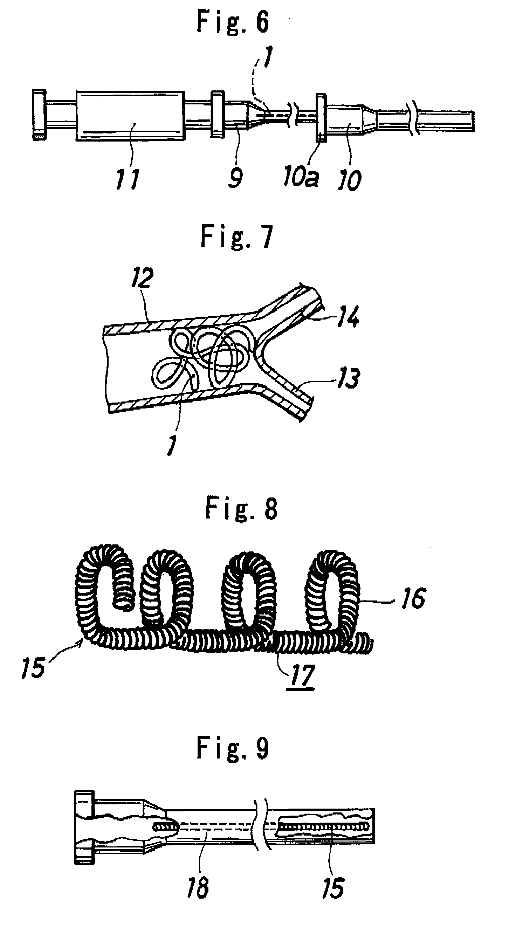

FIG. 8 shows a conventional vasoocclusive article made by winding a wire into a helical form of small diameter to obtain a primary helical coil 16, further winding the primary helical. coil into a secondary helical coil 17 of larger diameter, and winding polyester or like fibers on the resulting coil.

This vasoocclusive article 15 is caused to lodge at the desired intravascular site by placing the article 15 into a coil housing device 18 first as shown in FIG. 9, then pressing the forward end of the device against the hub cavity inside portion of a catheter as inserted into a blood vessel, inserting a guide wire into the housing device 18 through a rear-end opening thereof, and advancing the article through the catheter by pushing to the desired position, where the article is forced out of the catheter for the article to lodge at the site (see the publication of JP-A No. 2003-38498).

Conventional vasoocclusive articles are made of a metal wire such as stainless steel wire, platinum wire or the like. Vasoocclusive metal articles are low in compatibility with the living body, difficult to use for patients with a metal allergy and likely to cause infants and patients with no resistance to develop a fever, and produce artifact images in MRI (magnetic resonance imaging) diagnosis, presenting difficulty in conducting diagnosis. The vasoocclusive article lodging in the blood vessel will disturb images due to halation when to be checked by computerized tomography, and is therefore difficult to accurately locate and to check to detect the shape thereof.

Furthermore, the vasoocclusive metal article remains in the living body semipermanently without changing even after serving its function. Accordingly, when the occluded vascular site is to be surgically removed later, the metal article becomes an obstacle, possibly raising difficulties in surgical excision. Moreover, a surgical operation becomes necessary again when the lodging occlusive article itself is to be removed, while since another vasoocclusive article can not be used additionally, a more limited range of therapeutic choices will result.

Additionally, the blood blocking time is not controllable when occlusive metal articles are used, while they are not usable for cases involving the need to shut down the vessel for a short period of time only.

Because the flow of blood is unable to deliver the occlusive metal article to the desired site, the article can not be transported to the site inaccessible by catheters.

### SUMMARY OF THE INVENTION

An object of the present invention is to provide a vasoocclusive article which is free of the problems described above.

The present invention provides a vasoocclusive article comprising a filamentous substance made from a bioabsorbable polymer, the vasoocclusive article being transportable to a desired site by a flow of blood to lodge at the site after being placed into a catheter and subsequently pushed out of the catheter into a blood vessel.

The filamentous substance making the occlusive article is preferably 0.01 to 1 mm, more preferably 0.04 to 0.5 mm, in diameter and preferably 1 to 40 cm, more preferably 5 to 30 cm, in length.

### BRIEF DESCRIPTION OF THE DRAWINGS

FIG. 1 is a front view showing a vasoocclusive article comprising a filamentous substance in the form of a single line;
FIG. 2 is a front view showing a helical vasoocclusive article;
FIG. 3 is a front view showing a vasoocclusive article comprising a filamentous substance having a coil portion at one end thereof;
FIG. 4 is a front view showing a vasoocclusive article comprising a filamentous substance having a blood flow blocking member at intermediate portions thereof;
FIG. 5 is a plan view showing a vasoocclusive article having a J-shaped curve at each of opposite ends thereof.
FIG. 6 is a front view showing the vasoocclusive article while it is being inserted into a catheter;
FIG. 7 is a front view showing the vasoocclusive article lodging at the desired site.
FIG. 9 is a front view showing a conventional vasoocclusive article; and
FIG. 9 is a front view showing the conventional vasoocclusive article while it is being inserted into a catheter.

### DETAILED DESCRIPTION OF THE INVENTION

First, a description will be given of bioabsorbable polymers useful for making the filamentous substance.

The preferred bioabsorbable polymers are aliphatic polyester bioabsorbable polymers. Examples of such polymers are poly(a-hydroxy acids) such as poly(glycolic acid) and poly (lactic acid) ; poly- ε -caprolactone, polydioxanone, etc. These aliphatic polyesters are generally 60 to 200° C in melting point, -60 to 100° C in glass transition point and about 100,000 to about 300,000 in weight average molecular weight. Also useful are copolymers comprising glycolic acid as the main component unit, copolymers comprising lactic acid as the main component unit, copolymers comprising ε -caprolactone as the main component unit, and copolymers comprising dioxanone as the main component unit. Such polymers may be used in mixture.

Among the aliphatic polyesters mentioned above, especially preferable are poly(lactic acid), poly(lactic acid)-poly(glycolic acid) mixture, poly(lactic acid)-polycaprolactone mixture, because these polymers are excellent in bioabsorbability and high in safety in vivo and because glycolic acid or lactic acid which results from the decomposition of such polymers is absorbable in vivo. It is also desirable to incorporate chitosan or manganese into the bioabsorbable polymer.

Poly(lactic acid) is preferred because this polymer is excellent in mechanical strength, has good fiber properties, and is low in cost, transparent and amenable to coloring. With respect to safety in the living body, poly (lactic acid) is not only safe itself but is advantageous in that there is no need to add a plasticizer to this polymer unlike conventional polymer materials. Especially desirable as poly (lactic acid) is poly(L-lactic acid) which comprises L-lactic acid units since this polymer is excellent in mechanical strength and affords desired fiber properties.

The poly (lactic acid) is not limited to homopolymers but may be a lactic acid copolymer comprising other monomer component which is copolymerizable with lactic acid monomer or lactide. Examples of such monomer components are dicarboxylic acids, polyhydric alcohols, hydroxycarboxylic aids, lactones, etc. which have at least two ester linkage forming functional groups.

Poly (lactic acid) can be prepared by a known process as disclosed, for example, in the publication of JP-A No. 1995-3861, publication of JP-A No. 1984-96123, and Proceedings of the Symposium on High Polymers in Japan, Vol. 44, pp. 3198-3199. Thus, the polymer can be prepared from lactic acid by direct dehydration condensation, or by the ring-opening polymerization of lactic acid cyclic dimer lactide. It is also desirable to permit an increased quantity of the monomer to remain in poly (lactic acid) or to add an agent for promoting absorption in vivo to the polymer in order to give poly(lactic acid) enhanced bioabsorbability.

It is desired to incorporate a visually recognizable metal powder (such as bismuth sulfate powder) and/or an X-ray contrast medium component (such as barium sulfate, gold powder, platinum powder or iodine contrast medium) into the material for making the filamentous substance, i.e., into the bioabsorbable polymer. This makes it possible to transport the occlusive article to the desired intravascular site reliably and to observe the process of treatment more accurately.

The shapes of bioabsorbable vasoocclusive articles of the present invention will be described next.

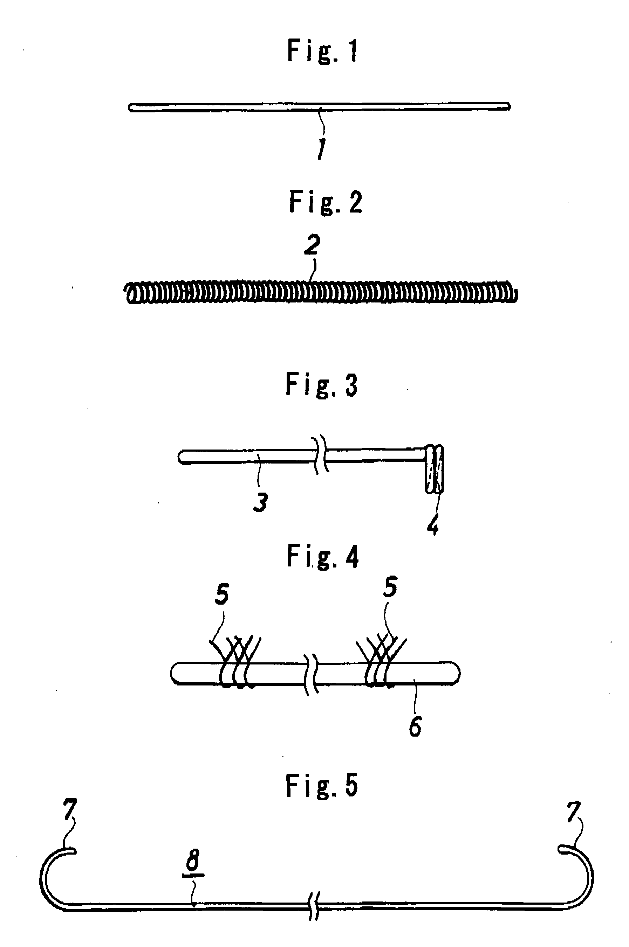

The vasoocclusive article of the present invention can be, for example, in the form of a filamentous substance resembling a single line and prepared from a bioabsorbable polymer (see FIG. 1), a helical occlusive article (see FIG. 2), a filamentous substance having a coil portion at one end thereof (see FIG. 3), a filamentous substance having a blood flow blocking member at a desired portion thereof (see FIG. 4), or a filamentous substance having a J-shaped curve at each of opposite ends thereof (see FIG. 5). The filamentous substance is preferably one having high flexibility, and can be a single filament but preferably comprise two or three filaments which are twisted.

When comprising a single filament of bioabsorbable polymer, the filamentous substance is prepared by extruding a composition of bioabsorbable polymer through the nozzle of an extruder. When comprising a twisted yarn of bioabsorbable polymer, the filamentous substance is obtained by twisting two or three of filaments thus prepared.

The helical occlusive substance is produced by helically winding a fibrous substance of bioabsorbable polymer around a straight mandrel, heat-treating the winding at a temperature approximate to the melting point of the polymer and removing the mandrel from the winding after cooling. The mandrel can be in the form of a metal wire. The mandrel is made of a material, such as stainless steel, copper or aluminum, having a higher melting point than the bioabsorbable polymer. The helical occlusive article is preferably 0.01 to 0.5 mm, more preferably 0.05 to 0.2 mm, in the diameter of helices.

To obtain the vasoocclusive article having a coil portion at one end thereof, the coil portion can be made by helically winding one end of a fibrous substance of bioabsorbable polymer around a straight mandrel, heat-treating the winding at a temperature approximate to the melting point of the polymer and removing the mandrel from the winding after cooling. The coil portion may be provided at one end of the fibrous substance or each end thereof. The diameter and the number of turns of the coil are determined in accordance with the symptoms.

With the vasoocclusive article having a blood flow blocking member, the blood flow blocking member preferably comprises a bundle of fibers of poly(lactic acid) or like bioabsorbable polymer. The polymer fiber bundle can be soft or hard, and a soft fiber bundle is usually used. The hard fiber bundle of bioabsorbable polymer is secured to a filamentous substance, for example, by thermal bonding. The length of the fiber bundle from the filamentous substance is preferably 1 to 10 mm. The blood flow blocking member is provided by winding the fiber bundle around the filamentous substance preferably at an intermediate portion of its length. The winding is preferably 2 to 8 mm in length.

A J-shaped curve can be given to opposite ends of a filamentous substance by placing a filamentous substance in the form of a single line into a die having a J-shaped groove, heat-treating the substance at a temperature close to the melting point thereof, or heat-treating the filamentous substance as held wound around a mandrel by one-half turn at each end thereof, and thereafter cooling the resulting substance. The vasoocclusive article thus obtained is delivered to the desired intravascular site of the patient for lodging. The article then restores the J-shaped curved ends although the curvature slightly increases.

How to use the bioabsorbable vasoocclusive article of the invention will be described next.

The vasoocclusive article of the invention is placed into a coil housing device first, the forward end of the device is then pressed against the hub cavity inside portion of a catheter as inserted into a blood vessel, and physiological saline solution is injected into the catheter through the coil housing device by a syringe to thereby advance the occlusive article inside the catheter to the desired position, where the occlusive article is pushed out of the catheter. The article pushed out is transported through the blood vessel by the flow of blood to the desired site (for example, a branch portion where a thick vessel is bifurcated into two smaller vessels), where the article becomes tangled to lodge.

The vasoocclusive article having a coil portion at the end of a filamentous substance can be delivered to the desired site promptly by the contact of the blood flow with the coil portion.

When lodging, the vasoocclusive article having a blood flow blocking member can effectively block the flow of blood.

The vasoocclusive article of the present invention consists mainly of a bioabsorbable polymer and is gradually decomposed as by hydrolysis to disappear when retained in the living body for a prolonged period of time. Although the period taken for disappearance varies with the conditions involved in the living body, the shape of the article, etc., the period can be controlled by selecting the proportion of the bioabsorbable material to be used.

It is desired that the bioabsorbable vasoocclusive article be filled with a carcinostatic, antitumor agent or like drug. This attains vasoocclusion and permits the drug to exhibit a sustained release effect. Alternatively, the article can be filled with an X-ray contrast medium component (barium sulfate, gold powder, platinum powder, iodine contrast medium or the like). Consequently, the occlusive article can be delivered to the desired intravascular site reliably, while the process of treatment can be observed with improved accuracy.
(1) The bioabsorbable vasoocclusive article of the present invention, which is made from a bioabsorbable material, has the following advantages.
   a) While lodging in the living body, the vasoocclusive article of the invention is gradually decomposed and no longer remains in vivo about the time when the article ceases functioning for vasoocclusion. The blood can therefore readily resume its flow, while another vasoocclusive article can be allowed to lodge additionally.
   b) The vasoocclusive article of the invention has good compatibility with the living body and is usable free of trouble for patients with a metal allergy or patients having no resistance.
   c) The duration of blood flow blocking is controllable by selecting the proportion of the bioabsorbable material. The article is therefore suitable to use when the flow of blood needs to be arrested temporarily as is the case with patients suffering a traffic accident.
   d) The vasoocclusive article produces no artifact images in MRI (magnetic resonance imaging) diagnosis, accordingly permits MRI diagnosis, causes no halation when to be checked by computerized tomography while lodging in the blood vessel and can therefore be accurately located and checked to detect the shape thereof.
(2) With the vasoocclusive article having a coil portion at one end of a filamentous substance, the coil portion serves to prevent the advance of the article into a smaller blood vessel because of its large diameter. This permits the selection of a particular blood vessel according to the diameter thereof. When placed into a blood vessel of large diameter, the article can be promptly transported to the desired site by the flow of blood in contact with the coil portion even when the flow of blood has low pressure.
(3) The vasoocclusive article having a J-shaped curve at opposite ends thereof is less likely to stimulate a thin-wall portion, for example, of aneurysm with its end, serving to reduce the burden on the patient.
(4) The vasoocclusive article having a blood flow blocking member effectively blocks the blood flow when lodging.
(5) The vasoocclusive article of the invention is transported to the desired site by the flow of blood to lodge at the site after being placed into a catheter and subsequently pushed out of the catheter into a blood vessel. Accordingly, the article can be transported to sites in accessible by the catheter.
(6) The bioabsorbable vasoocclusive article can be filled with a carcinostatic, antitumor agent or like drug. This attains vasoocclusion and permits the drug to exhibit a sustained release effect. When filled with a visually recognizable metal powder (such as bismuth sulfate powder) and/or an X-ray contrast medium component (such as barium sulfate, gold powder, platinum powder or iodine contrast medium), the occlusive article can be delivered to the desired intravascular site reliably, while the process of treatment can be observed with improved accuracy.

The present invention will be described in greater detail with reference to the following examples, which nevertheless in no way limit the invention.

### [Example 1]

Poly(L-lactic acid) (product of Shimadzu Corp., trade name "Lacty," 200,000 in weight average molecular weight, 175° C in melting point) was melt-spun at 200° C and drawn to obtain a single filament, 0.05 mm in diameter. Two lengths of the filament were twisted to prepare a filamentous substance, which was cut to a length of 7 cm. In this way, vasoocclusive article 1 in the form of a single line and shown in FIG. 1 was made. The filamentous substance had sufficient flexibility and was itself usable (in the form of a single line) as a vasoocclusive article.

### [Example 2]

The filamentous substance obtained in Example 1 was helically wound around a straight stainless steel mandrel having a diameter of 0.2 mm, heat-treated at 150° C, cooled and thereafter removed from the mandrel. In this way, a helical vasoocclusive article 2 was prepared which was in the form of a helical winding shown in FIG. 2 and having a diameter of 0.4 mm and a length of about 5 cm in length.

### [Example 3]

One end portion of the filamentous substance obtained in Example 1 was helically wound around a straight mandrel, heat-treated at a temperature close to the melting point of the polymer, cooled and thereafter removed from the mandrel. In this way, a vasoocclusive article 3 was prepared which is shown in FIG. 3 and was provided at one end thereof with a coil portion 4 having a diameter of 0.4 mm and two turns of winding.

### [Example 4]

A bundle of soft fibers of poly(lactic acid) was wound around the filamentous substance obtained in Example 1 at each of intermediate portions thereof. In this way, a vasoocclusive article 6 was prepared which is shown in FIG. 4 and had blood flow blocking members 5 each measuring 4 mm in winding length and 2 mm in length from the filamentous substance.

### [Example 5]

Opposite end portions of the filamentous substance obtained in Example 1 were wound around a stainless steel mandrel having a diameter of 1 mm one-half turn, each held curled in a J-shaped form, heat-treated at 150° C and thereafter cooled. In this way, a vasoocclusive article 8 was prepared which is shown in FIG. 5 and had a J-shaped curve portion 7 at each end thereof.

### [Example 6]

A vasoocclusive article in the form of a single line was prepared in the same manner as in Example 1 except that a carcinostatic was incorporated into the poly(L-lactic acid).

### [Example 7]

A vasoocclusive article in the form of a single line was prepared in the same manner as in Example 1 except that a bismuth sulfate was incorporated into the poly(L-lactic acid).

### [Method of Use]

With reference to FIG. 6, the vasoocclusive article 1 obtained in Example 1 is placed into a coil housing device 9 first, the forward end of the device 9 is then pressed against the hub cavity inside portion 10a of a catheter 10 as inserted into a blood vessel, and physiological saline solution is injected into the catheter 10 through the coil housing device 9 by a syringe 11 to thereby advance the occlusive article 1 inside the catheter 10 to the desired position, where the occlusive article 1 is pushed out of the catheter 10. The article 1 pushed out is transported through the blood vessel by the flow of blood to the desired site (for example, a branch portion where a thick vessel 12 is bifurcated into two smaller vessels 13, 14), where the article 1 becomes tangled to lodge as shown in FIG. 7.

## Claims

1. A vasoocclusive article comprising a filamentous substance made from a bioabsorbable polymer, the vasoocclusive article being transportable to a desired site by a flow of blood to lodge at the site after being placed into a catheter and subsequently pushed out of the catheter into a blood vessel.

2. A vasoocclusive article according to claim 1 which is in a helical form.

3. A vasoocclusive article according to claim 1 which has a coil portion at at least one end thereof.

4. A vasoocclusive article according to claim 1 which has a blood flow blocking member provided at a desired portion thereof.

5. A vasoocclusive article according to claim 1 which has a J-shaped curve at at least one end portion thereof.

6. A vasoocclusive article according to claim 1 which has a visually recognizable metal powder incorporated therein.

7. A vasoocclusive article according to claim 1 wherein the bioabsorbable polymer is poly (lactic acid), a poly (lactic acid)-poly(glycolic acid) mixture or a poly(lactic acid)-polycaprolactone mixture.

8. A vasoocclusive article according to claim 1 wherein the filamentous substance is 0.01 to 1 mm in diameter.

9. A vasoocclusive article according to claim 1 wherein the filamentous substance is 1 to 40 cm in length.
